# EUROPÄISCHE PATENTSCHRIFT

(11) **EP 3 037 057 B1**
(45) Veröffentlichungstag und Bekanntmachung des Hinweises auf die Patenterteilung: **02.11.2016**
(21) Anmeldenummer: 14199570.4
(22) Anmeldetag: 22.12.2014
(51) Int. Cl.: A61C 1/00, A61C 1/10, A61C 1/12, A61C 1/07

(54) **MEDIZINISCHES, INSBESONDERE ZAHNÄRZTLICHES, HAND- ODER WINKELSTÜCK**
Medical, in particular dental, straight or contra-angle handpiece
Pièce à main ou pièce coudée médicale, en particulier dentaire

(43) Veröffentlichungstag der Anmeldung: 29.06.2016
(73) Patentinhaber: W & H Dentalwerk Bürmoos GmbH, 5111 Bürmoos (AT)
(72) Erfinder: Mangelberger, Michael, 5113 St. Georgen (AT); Pointner, Michael, 5111 Bürmoos (AT)

(56) Entgegenhaltungen:
- EP-A2- 0 323 598
- EP-A2- 2 664 288
- WO-A2-01/54611

## Beschreibung

Die vorliegende Erfindung bezieht sich auf medizinisches, insbesondere zahnärztliches, Hand- oder Winkelstück zum Antrieb eines medizinischen, insbesondere zahnärztlichen, Werkzeugs nach dem Oberbegriff des Anspruchs 1 sowie auf eine medizinische, insbesondere zahnärztliche, Behandlungsvorrichtung nach dem Oberbegriff des Anspruchs 15.

Derartige medizinische Hand- oder Winkelstücke dienen bevorzugt zur Bearbeitung von hartem oder weichem Gewebe. Hierzu werden medizinische, insbesondere zahnärztliche, Werkzeuge bevorzugt durch eine mit dem Hand- oder Winkelstück bevorzugt lösbar verbindbare Antriebseinheit angetrieben. Die Antriebseinheiten weisen hierbei bevorzugt einen Elektromotor oder einen Piezoantrieb auf. Zur Übertragung der Antriebsbewegung von der Antriebseinheit auf die Werkzeuge weisen die Hand- oder Winkelstücke einen Antriebsstrang mit zumindest einer in Rotation und/ oder Schwingung versetzbaren Welle auf, so dass die Werkzeuge in eine Arbeitsbewegung versetzbar sind. Während des Betriebs der medizinischen Hand- oder Winkelstücke sind diese sowie die damit bevorzugt lösbar verbundenen Antriebseinheiten mit Arbeitsmedien, wie zum Beispiel elektrischer Energie, zu versorgen. Diese Medien werden bevorzugt von einer zahnärztlichen Einheit bereit gestellt und mittels eines Versorgungsschlauchs der Antriebseinheit und dem Hand- oder Winkelstück zugeführt.

Zur Vermeidung einer Erwärmung des zu behandelnden Gewebes, der verwendeten medizinischen Werkzeuge sowie der medizinischen Hand- oder Winkelstücke ist es nun bekannt die medizinischen Hand- oder Winkelstücke mit Fluidleitungen zu versehen, durch die ein Kühlmedium, insbesondere Fluide, Gase oder Gasgemische, förderbar ist. Die Fluidleitungen erstrecken sich hierbei bevorzugt durch die medizinischen Hand- oder Winkelstücke und enden im Kopfbereich der Hand- oder Winkelstücke, welcher zur Aufnahme von medizinischen Werkzeugen ausgebildet ist. Durch bevorzugt mehre Austrittsöffnungen in dem Handstückkopf tritt das Kühlmedium aus den Fluidleitungen aus und gelangt so auf das im Hand- oder Winkelstück aufgenommenen Werkzeug und auf die Behandlungsstelle. Hierdurch ist gleichzeitig das Hand- oder Winkelstück, das Werkzeug und der Arbeitsbereich kühlbar.

Bei einer chirurgischen und implantologischen Anwendung hingegen erfolgt eine Kühlung der Behandlungsstelle und des Werkzeugs durch die Zuführung eines Kühlmittels, insbesondere von Wasser oder einer Kochsalzlösung, mittels einer sogenannten Außenkühlung. Hierbei wird das Kühlmittel entlang der Außenseite des Handstücks in einer externen Fluidleitung bis zum Handstückkopf und somit bis zu dem im Hand- oder Winkelstück aufgenommenen Werkzeug geführt. Die Abgabe des Kühlmittels erfolgt auf das Werkzeug und auf die Behandlungsstelle. Hierdurch wird eine Verunreinigung des Kühlmediums durch mögliche Verschmutzungen in den Hand- oder Winkelstücken vermieden.

Als nachteilig dieser Ausgestaltung der Hand- oder Winkelstücke erweist sich hierbei jedoch, dass keine Kühlung der Hand- oder Winkelstücke erfolgt. Durch die fehlende Kühlung ist eine Erwärmung der medizinischen Hand- oder Winkelstücke unvermeidbar, wodurch die Gefahr von Verbrennung bei dem Anwender und dem Patienten auf Grund einer Überhitzung der Hand- oder Winkelstücke besteht.

Des Weiteren können Beschädigungen an den Hand- oder Winkelstücken, insbesondere an deren Antriebssträngen, auftreten. Die mechanischen Bauteile der Antriebsstränge, insbesondere die Wellen und Kugellager, welche bevorzugt zur Übertragung einer rotierenden Antriebsbewegung dienen, sind in regelmäßigen Abständen zu schmieren. Bei eine fehlenden Schmierung, zum Beispiel auf Grund einer Überhitzung des Hand- oder Winkelstücks und der damit verbundenen Zersetzung des Schmiermittels, besteht somit die Gefahr einer Beschädigung der Antriebsstränge.

Des Weiteren hat eine Erwärmung der mit den medizinischen Hand- oder Winkelstücken verbindbaren Antriebseinheiten eine Leistungsminderung insbesondere der in den Antriebseinheiten angeordneten Elektromotoren zur Folge. Hierdurch ist ebenfalls die medizinische, insbesondere zahnärztliche, Behandlung gefährdet.

Der vorliegenden Erfindung liegt daher die Aufgabe zugrunde ein medizinisches, insbesondere zahnärztliches, Hand- oder Winkelstück zum Antrieb eines medizinischen, insbesondere zahnärztlichen, Werkzeugs zu schaffen, das es ermöglicht die Nachteile des Stand der Technik zur vermeiden und gleichzeitig bei der Präparation von Zähnen als auch bei der chirurgischen und implantologischen Anwendung einsetzbar ist.

Gemäß einem Ausführungsbeispiel eines medizinischen, insbesondere zahnärztlichen, Hand- oder Winkelstücks zum Antrieb eines medizinischen, insbesondere zahnärztlichen, Werkzeugs, weist dieses ein erstes Kupplungselement zur bevorzugt lösbaren Verbindung des Hand- oder Winkelstücks mit einer Antriebseinheit zur Übertragung einer Antriebsbewegung und eines Kühlmediums, eine Werkzeugaufnahme zum bevorzugt lösbaren Anschließen des medizinischen, insbesondere zahnärztlichen, Werkzeugs sowie einen Antriebsstrang zur Übertragung einer Antriebsbewegung von der Antriebseinheit auf die Werkzeugaufnahme auf, welcher zumindest eine in Rotation und/ oder Schwingung versetzbare Welle umfasst, wobei das erste Kupplungselement zur Kühlung des Hand- oder Winkelstücks zumindest einen ersten Fluideinlass und zumindest einen ersten Fluidauslass umfasst und wobei der zumindest eine erste Fluideinlass und der zumindest eine erste Fluidauslass durch zumindest eine erste sich durch das Hand- oder Winkelstück erstreckende Fluidleitung verbunden sind, so dass ein Kühlmedium von dem zumindest einen ersten Fluideinlass durch die Fluidleitung zu dem zumindest einen ersten Fluidauslass förderbar ist.

Gemäß einem ersten Ausführungsbeispiel des medizinischen, insbesondere zahnärztlichen, Hand- oder Winkelstücks umfasst das Hand- oder Winkelstück zumindest eine zweite sich durch das Hand- oder Winkelstück erstreckende Fluidleitung mit zumindest einem zweiten Fluideinlass und zumindest einem zweiten Fluidauslass, wobei der zumindest eine zweite Fluideinlass und der zumindest eine zweite Fluidauslass an dem ersten Kupplungselement und/ oder an der zumindest einen ersten Fluidleitung des medizinischen Hand- oder Winkelstücks angeordnet sind.

Hierbei umfasst die erste und/ oder zweite Fluidleitung des Hand- oder Winkelstücks zumindest ein Rückschlagventil, so dass ein erstes und zweites Kühlmedium in der ersten und zweiten Fluidleitung nicht in die jeweils andere Fluidleitung des Hand- oder Winkelstücks gelangt.

Gemäß einem zweiten Ausführungsbeispiel des medizinischen, insbesondere zahnärztlichen, Hand- oder Winkelstücks sind die zumindest eine erste und zweite Fluidleitung durch ein Leitungsrohr, ein den Antriebsstrang aufnehmendes Gehäuse, eine Bohrung in dem Gehäuse und/ oder durch das erste Kupplungselement gebildet.

Gemäß einem dritten Ausführungsbeispiel des medizinischen, insbesondere zahnärztlichen, Hand- oder Winkelstücks sind die zumindest eine erste und zweite Fluidleitung hermetisch abgeschlossen, so dass das erste und zweite Kühlmedium nicht aus dem Hand- oder Winkelstück austritt.

Gemäß einem vierten Ausführungsbeispiel des medizinischen, insbesondere zahnärztlichen, Hand- oder Winkelstücks umgibt die erste und/ oder zweite Fluidleitung die Werkzeugaufnahme des Hand- oder Winkelstücks zumindest teilweise, so dass diese kühlbar ist.

Gemäß einem fünften Ausführungsbeispiel des medizinischen, insbesondere zahnärztlichen, Hand- oder Winkelstücks weist das Hand- oder Winkelstück zumindest eine bevorzugt lösbar mit dem Hand- oder Winkelstück verbindbare Antriebseinheit auf, welche mit einem Versorgungsschlauch zur Übertragung von Daten und/ oder Energie sowie zumindest einem Kühlmedium verbindbar ist und zumindest eine erste durch die Antriebseinheit verlaufende Fluidleitung und zumindest eine zweite von der ersten Fluidleitung getrennte Fluidleitung umfasst, wobei die erste und zweite Fluidleitung der Antriebseinheit über den zumindest einen ersten Fluideinlass und den zumindest einen ersten Fluidauslass des Hand- oder Winkelstücks mit der zumindest einen ersten Fluidleitung des Hand- oder Winkelstücks verbindbar sind, so dass ein Kühlmedium von der ersten Fluidleitung der Antriebseinheit über die sich durch das Hand- oder Winkelstück erstreckende Fluidleitung in die zweite Fluidleitung der Antriebseinheit förderbar ist.

Gemäß einem weiteren Ausführungsbeispiel des medizinischen, insbesondere zahnärztlichen, Hand- oder Winkelstücks weist die Antriebseinheit ein zweites Kupplungselement auf, welches bevorzugt lösbar mit dem ersten Kupplungselement des Hand- oder Winkelstücks verbindbar ist, wobei eines der beiden Kupplungselemente als Kupplungsausnehmung ausgebildet ist, in die ein Kupplungsvorsprung des anderen Kupplungselements einsetzbar ist.

Hierbei sind der zumindest eine erste Fluideinlass und der zumindest eine erste Fluidauslass des Hand- oder Winkelstücks bevorzugt in dem Kupplungsvorsprung oder der Kupplungsausnehmung angeordnet.

Gemäß einem weiteren Ausführungsbeispiel des medizinischen, insbesondere zahnärztlichen, Hand- oder Winkelstücks umfasst die Antriebseinheit einen Elektromotor mit einer Rotoreinheit, welche bevorzugt drehbar in einem Statorpaket gelagert ist. Alternativ kann die Antriebseinheit auch einen Luftmotor oder einen Piezoantrieb aufweisen.

Gemäß allen voranstehenden Ausführungsbeispielen des medizinischen, insbesondere zahnärztlichen, Hand- oder Winkelstücks ist der zumindest eine durch die mehreren Fluidleitungen, Fluideinlässe und Fluidauslässe gebildete Fluidkreislauf hermetisch abgeschlossen, so dass das Kühlmedium nicht aus dem Hand- oder Winkelstück und/ oder der Antriebseinheit austritt.

Zusätzlich steigen gemäß allen voranstehenden Ausführungsbeispielen bevorzugt die Querschnitte der mehreren Fluidleitungen in Richtung des Fluidstromes an, so dass ein Rückstau des zumindest einen Kühlmediums in dem zumindest einen Fluidkreislauf vermeidbar ist.

Des Weiteren weisen gemäß allen voranstehenden Ausführungsbeispielen die Fluideinlässe und die Fluidauslässe der Fluidleitungen des Hand- oder Winkelstücks bevorzugt jeweils einen Regelungsabschnitt zur Regelung eines Fluidstroms auf, so dass in einem entkoppelten Zustand beider Kupplungselemente ein in den Fluidleitungen verbleibendes Kühlmedium nicht aus dem Hand- oder Winkelstück und/ oder der Antriebseinheit strömbar ist.

Der Regelungsabschnitt ist hierzu bevorzugt durch ein Rückschlagventil ausgebildet, welches einen Ventilsitz und einen Regelungskörper aufweist.

Gemäß einem Ausführungsbeispiel einer medizinischen, insbesondere zahnärztlichen, Behandlungsvorrichtung zum Antrieb eines medizinischen, insbesondere zahnärztlichen, Werkzeugs weist diese zumindest ein medizinisches, insbesondere zahnärztliches, Hand- oder Winkelstück nach einem der vorherigen Ausführungsbeispiele, zumindest einen mit dem Hand- oder Winkelstück bevorzugt lösbar verbindbaren Versorgungsschlauch zur Übertragung zumindest eines Kühlmediums, sowie zumindest eine Steuereinheit zum Antreiben und/ oder Ansteuern des zumindest einen Hand- oder Winkelstücks auf, welche dazu ausgebildet ist den Fluidkreislauf des Hand- oder Winkelstücks mit einer Medienquelle und einen Medienauslass zu verbinden, so dass das Hand- oder Winkelstück von einem Kühlmedium durchströmbar ist.

Das vorliegende medizinische, insbesondere zahnärztliche, Hand- oder Winkelstück zeichnet sich durch eine Reihe erheblicher Vorteile aus.

Durch die Anordnung zumindest eines Fluideinlasses und zumindest eines Fluidauslasses an dem Kupplungselement des Hand- oder Winkelstücks, welche durch zumindest eine durch das Hand- oder Winkelstück erstreckende Fluidleitung verbunden sind, ist ein Kühlmedium durch das Hand- oder Winkelstück förderbar. Hierdurch ist das medizinische Hand- oder Winkelstück separat von dem in dem Hand- oder Winkelstück aufgenommenen Werkzeug und der Behandlungsstelle kühlbar. Dadurch wiederum ist das Hand- oder Winkelstück, insbesondere mit der Antriebseinheit, gleichzeitig bei der Präparation von Zähnen als auch bei der chirurgischen und implantologischen Anwendung einsetzbar.

Des Weiteren ergeben sich folgende wesentlichen Vorteile:
Durch die Kühlung des Hand- oder Winkelstücks wird die Gefahr von möglichen Verbrennungen auf Grund einer Überhitzung des Hand- oder Winkelstücks bei Anwender und Patient reduziert.

Beschädigungen an dem Hand- oder Winkelstück, insbesondere an dessen Antriebsstrang, auf Grund von zersetzten Schmiermittels, können ebenso vermieden werden.

Des Weiteren wird durch eine Kühlung des Hand- oder Winkelstücks eine Leistungsminderung bei dem medizinischen Hand- oder Winkelstück, insbesondere bei dem in der Antriebseinheit des Hand- oder Winkelstücks angeordneten Elektromotors, vermieden, was eine sichere medizinische, insbesondere zahnärztliche, Behandlung gewährleistet.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel des medizinischen, insbesondere zahnärztlichen, Hand- oder Winkelstücks,
Figur 2 ein zweites Ausführungsbeispiel des medizinischen, insbesondere zahnärztlichen, Hand- oder Winkelstücks mit einer bevorzugt lösbar mit dem Hand- oder Winkelstück verbindbaren Antriebseinheit und
Figur 3 eine medizinische, insbesondere zahnärztliche, Behandlungsvorrichtung mit zumindest einem medizinischen Hand- oder Winkelstück, einem Versorgungsschlauch zur Übertragung zumindest eines Kühlmediums, sowie zumindest einer Steuereinheit zum Antreiben und/ oder Ansteuern des zumindest einen Hand- oder Winkelstücks;

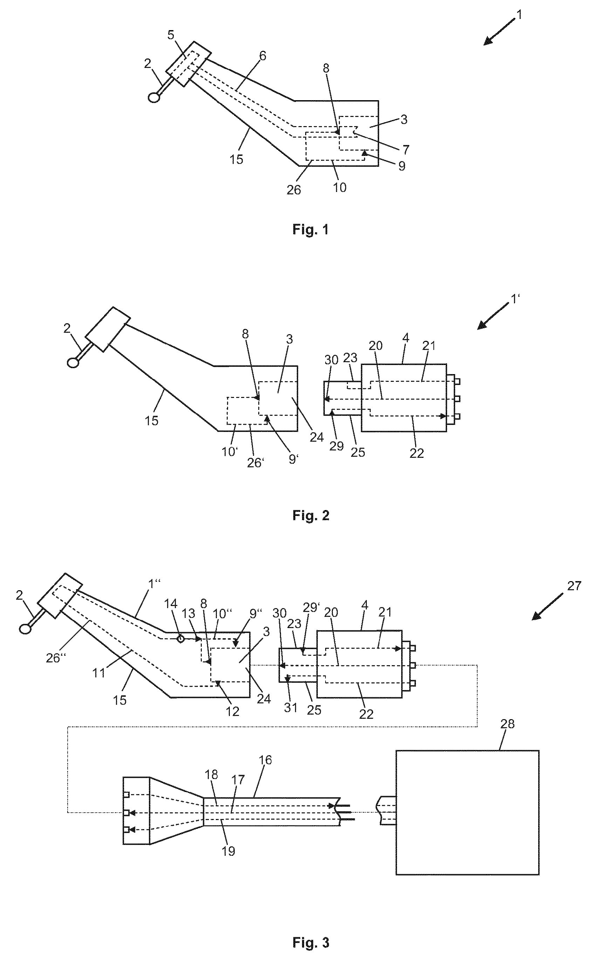

Das in Figur 1 dargestellte medizinische, insbesondere zahnärztliche Hand- oder Winkelstück 1 zum Antrieb eines medizinischen, insbesondere zahnärztlichen, Werkzeugs 2 umfasst eine Werkzeugaufnahme 5 zum bevorzugt lösbaren Anschließen des medizinischen Werkzeugs 2. Die Werkzeugaufnahme 5 ist hierbei bevorzugt als Spannzange ausgebildet, welche durch einen Druckknopf betätigbar ist. In dem Hand- oder Winkelstück 1 ist ein Antriebsstrang 6 zur Übertragung einer Antriebsbewegung von einer Antriebseinheit auf die Werkzeugaufnahme 5, welcher zumindest eine in Rotation versetzbare Welle 7 umfasst, angeordnet. Die zumindest eine Welle 7 ist hierbei bevorzugt mittels mehrerer Kugellager drehbar in dem Hand- oder Winkelstück 1 gelagert. In dem gezeigten Ausführungsbeispiel umfasst der Antriebsstrang 6 bevorzugt zumindest zwei Wellen, welche gewinkelt zueinander in dem Gehäuse 15 des Hand- oder Winkelstücks 1 angeordnet sind.

Zur bevorzugt lösbaren Verbindung des Hand- oder Winkelstücks 1 mit einer Antriebseinheit und zur Übertragung einer Antriebsbewegung und eines Kühlmediums weist das Hand- oder Winkelstück 1 ein erstes Kupplungselement 3 auf. Die erste Welle 7 des Antriebsstrangs 6 ragt hierbei bevorzugt in das erste Kupplungselement 3. Das erste Kupplungselement 3 selbst umfasst hierbei zumindest einen ersten Fluideinlass 8 und zumindest einen ersten Fluidauslass 9, welche durch zumindest eine erste sich durch das Hand- oder Winkelstück 1 erstreckende Fluidleitung 10 verbunden sind, so dass ein Kühlmedium, insbesondere ein Fluid, Gas oder Gasgemisch, von dem zumindest einen ersten Fluideinlass 8 durch die Fluidleitung 10 zu dem zumindest einen ersten Fluidauslass 9 förderbar ist und das Hand- oder Winkelstück 1 kühlbar ist. Die zumindest eine Fluidleitung 10 ist hierbei bevorzugt durch das den Antriebsstrang 6 aufnehmende Gehäuse 15 sowie durch eine Bohrung in dem Gehäuse 15 und des ersten Kupplungselements 3 gebildet und in dem Gehäuse 15 des Hand- oder Winkelstücks 1 hermetisch abgeschlossen. Die Fluidleitung 10 ist ein durchgängiges Bauteil ohne Öffnungen.

Die zumindest eine Fluidleitung 10, der erste Fluideinlass 8 sowie der erste Fluidauslass 9 in dem ersten Kupplungselement 3 des Hand- oder Winkelstücks 1 bilden somit in dem Hand- oder Winkelstück 1 einen hermetisch abgeschlossenen Fluidkreislauf 26, welcher von einem Kühlmedium durchströmbar ist. Das Kühlmedium wird hierbei an dem rückwärtigen Ende des Hand- oder Winkelstücks 1 in das Hand- oder Winkelstück 1 geleitet, zirkuliert in dem Hand- oder Winkelstück 1, insbesondere in dessen Gehäuse 15, und tritt an dessen rückwärtigen Ende, insbesondere an dem ersten Kupplungselement 3, wieder aus. Hierdurch ist es möglich das medizinische Hand- oder Winkelstück 1 zu kühlen, ohne dass das Kühlmedium in den Arbeitsbereich des medizinischen Werkzeugs 2 gelangt. Alternativ ist es auch denkbar, dass zu Beginn einer Behandlung das Hand- oder Winkelstück 1, insbesondere der Fluidkreislauf 26, mit einem vorgewärmten Medium durchströmt wird bis das Hand- oder Winkelstück 1 eine bevorzugt vorherbestimmte Betriebstemperatur erreicht hat.

In Figur 2 ist ein medizinisches Hand- oder Winkelstück 1' zum Antrieb eines medizinischen Werkzeugs 2 abgebildet, welches zumindest eine bevorzugt lösbar mit dem Hand- oder Winkelstück 1' verbindbare Antriebseinheit 4 aufweist. Die Antriebseinheit 4 wiederum ist mit einem Versorgungsschlauch zur Übertragung von Daten und/ oder Energie sowie zumindest einem Kühlmedium verbindbar. Die Antriebseinheit 4 umfasst hierbei bevorzugt einen Elektromotor mit einer Rotoreinheit, welche bevorzugt drehbar in einem Statorpaket gelagert ist. Des Weiteren weist die Antriebseinheit 4 ein zweites Kupplungselement 23 auf, welches bevorzugt lösbar mit dem ersten Kupplungselement 3 des Hand- oder Winkelstücks 1' verbindbar ist. Das Kupplungselement 3 des Hand- oder Winkelstücks 1' ist hierbei bevorzugt als Kupplungsausnehmung 24 ausgebildet ist, in die ein Kupplungsvorsprung 25 der Antriebseinheit 4 einsetzbar ist.

Zur Kühlung des Hand- oder Winkelstücks 1' weist die Antriebseinheit 4 zumindest eine erste durch die Antriebseinheit 4 verlaufende Fluidleitung 20 und zumindest eine zweite von der ersten Fluidleitung 20 getrennte Fluidleitung 22 auf. Beide Fluidleitungen 20, 22 der Antriebseinheit 4 sind über den zumindest einen ersten Fluideinlass 8 und den zumindest einen ersten Fluidauslass 9' des Hand- oder Winkelstücks 1' mit der zumindest einen ersten Fluidleitung 10' des Hand- oder Winkelstücks 1' verbindbar. Hierzu umfassen die Fluidleitungen 20, 22 der Antriebseinheit 4 bevorzugt einen Fluideinlass 29 und einen Fluidauslass 30, welche bevorzugt in dem Kupplungsvorsprung 25 der Antriebseinheit 4 angeordnet sind und mit dem zumindest einen Fluideinlass 8 und dem zumindest einen Fluidauslass 9' in der Kupplungsausnehmung 24 des Hand- oder Winkelstücks 1' koppelbar sind. Ein Kühlmedium ist so von der ersten Fluidleitung 20 der Antriebseinheit 4 über die sich durch das Hand- oder Winkelstück 1' erstreckende Fluidleitung 10' in die zweite Fluidleitung 22 der Antriebseinheit 4 förderbar.

Auch in diesem Ausführungsbeispiel sind die mehreren Leitungen 10', 20, 22 in dem Hand- oder Winkelstück 1' und der Antriebseinheit 4, insbesondere der durch die Leitungen 10', 20, 22 sowie durch die mehreren Fluideinlässe 8, 29 und Fluidauslässe 9', 30 gebildete Fluidkreislauf 26' in dem Gehäuse 15 des Hand- oder Winkelstücks 1' hermetisch abgeschlossen, so dass das Kühlmedium in dem Hand- oder Winkelstück 1' zirkulierbar ist und nicht aus dem Gehäuse 15 des Hand- oder Winkelstücks 1' und der Antriebseinheit 4 austreten kann.

In dem gezeigten Ausführungsbeispiel gemäß Figur 2 umfasst die Antriebseinheit 4 des Hand- oder Winkelstücks 1' neben den Leitungen 20, 22 eine dritte Fluidleitung 21. Hierdurch ist es möglich, dass die Antriebseinheit 4 auch bei zahnärztlichen Behandlungen, insbesondere bei der Bearbeitung von Zähnen, verwendbar ist, bei denen Hand- oder Winkelstücke eingesetzt werden, welche eine Kühlung des Hand- oder Winkelstücks, des Werkzeug und des Arbeitsbereiches mittels mehrere Kühlmedium, insbesondere mittels Wasser und Luft, ermöglichen. Die Kühlmedien werden hierbei durch die Hand- oder Winkelstücke geführt und treten im Kopfbereich aus den Hand- oder Winkelstücken aus.

Die mehreren Fluidleitungen 20, 21, 22 der Antriebseinheit 4 sind bevorzugt durch ein Leitungsrohr in der Antriebseinheit 4 und/ oder durch einen Freiraum zwischen dem bevorzugt in der Antriebseinheit 4 angeordneten Elektromotor und dem Gehäuse der Antriebseinheit 4 gebildet. Des Weiteren steigen die Querschnitte der Fluidleitungen 10', 20, 22 bevorzugt in Fluidrichtung des Kühlmediums an, um einen Rückstau des Kühlmediums in dem zumindest einen Fluidkreislauf 26' des Hand- oder Winkelstücks 1' zu vermeiden.

Damit auch in einem entkoppelten Zustand beider Kupplungselemente 3, 23 ein in den Fluidleitungen 10', 20, 22 verbleibendes Kühlmedium nicht aus dem Hand- oder Winkelstück 1' und der Antriebseinheit 4 strömbar ist, weisen die Fluideinlässe 8, 29 und Fluidauslässe 9', 30 der Fluidleitungen 10', 20, 22 bevorzugt jeweils einen Regelungsabschnitt zur Regelung eines Fluidstroms auf. Diese sind bevorzugt durch selbstschließende Rückschlagventil ausgebildet, welche einen Ventilsitz und einen Regelungskörper aufweisen. Sobald die Fluidleitungen 10', 20, 22 miteinander gekuppelt sind und/ oder mit einem Kühlmedium beaufschlagt werden, öffnen diese Ventile.

In Figur 3 ist eine medizinische, insbesondere zahnärztliche, Behandlungsvorrichtung 27 zum Antrieb eines medizinischen, insbesondere zahnärztlichen, Werkzeugs 2 gezeigt. Die Behandlungsvorrichtung 27 umfasst hierbei ein medizinisches, insbesondere zahnärztliches, Hand- oder Winkelstück 1", einen mit dem Hand- oder Winkelstück 1" bevorzugt lösbar verbindbaren Versorgungsschlauch 16 zur Übertragung zumindest eines Kühlmediums sowie zumindest eine Steuereinheit 28 zum Antreiben und/ oder Ansteuern des zumindest einen Hand- oder Winkelstücks 1".

Das medizinische Hand- oder Winkelstück 1" weist auch in diesem Ausführungsbeispiel eine bevorzugt lösbar mit dem Hand- oder Winkelstück 1" verbindbare Antriebseinheit 4 auf, welche mittels eines zweiten Kupplungselements 23 bevorzugt lösbar mit dem ersten Kupplungselement 3 des Hand- oder Winkelstücks 1" verbindbar ist.

Neben dem ersten Fluideinlass 8 und dem ersten Fluidauslass 9" in der Kupplungsausnehmung 24 des Hand- oder Winkelstücks 1", welche durch zumindest eine erste sich durch das Hand- oder Winkelstück 1" erstreckende Fluidleitung 10" verbunden sind, weist das Hand- oder Winkelstück 1" einen zweiten Fluideinlass 12 auf. Dieser ist mit einer zweiten sich durch das Hand- oder Winkelstück 1" erstreckenden Fluidleitung 11 gekoppelt. Die zweite Fluidleitung 11 erstreckt sich hierbei bevorzugt bis in den Kopfbereich des Hand- oder Winkelstücks 1" und umgibt die dort angeordnete Werkzeugaufnahme 5 zumindest teilweise, so dass diese kühlbar ist. Die zweite Fluidleitung 11 endet mittels eines zweiten Fluidauslasses 13 in der ersten Fluidleitung 10" des Hand- oder Winkelstücks 1". Damit ein erstes Kühlmedium in der ersten Fluidleitung 10" nicht in die zweite Fluidleitung 11 gelangt, weist die zweite Fluidleitung 11 bevorzugt zumindest ein Rückschlagventil 14 auf. Beide Fluidleitungen 10", 11 sind auch in diesem Ausführungsbeispiel bevorzugt durch ein den Antriebsstrang aufnehmendes Gehäuse 15 sowie durch zumindest eine Bohrung in dem Gehäuse 15 und in dem Kupplungselement 3 gebildet.

Zum Fördern zumindest zweier Kühlmedien durch das Hand- oder Winkelstück 1" weist die Antriebseinheit 4 drei durch die Antriebseinheit 4 verlaufende Fluidleitungen 20, 21 und 22 auf. Die Fluidleitungen 20 und 22 sind hierbei zum Zuführen zumindest zweier Kühlmedien über die in dem Kupplungsvorsprung 25 der Antriebseinheit 4 angeordneten Fluidauslässe 30, 31 und den in dem Hand- oder Winkelstück 1" angeordneten Fluideinlässen 8, 12 mit der ersten und zweiten Fluidleitung 10", 11 des Hand- oder Winkelstücks 1" verbindbar. Zur Rückführung der Kühlmedien ist der Fluidauslass 9" der Fluidleitung 10" des Hand- oder Winkelstücks 1" mit dem Fluideinlass 29' der sich durch die Antriebseinheit 4 erstreckenden Fluidleitung 21 koppelbar. Die Kühlmedien sind so durch die Antriebseinheit 4 und durch das Hand- oder Winkelstück 1" förderbar. Des Weiteren ist es dadurch möglich, das medizinische Hand- oder Winkelstück 1" und die Antriebseinheit 4 mittels zweier Kühlmedien separat zu kühlen, ohne dass eines der beiden Kühlmedien in den Arbeitsbereich des medizinischen Werkzeugs 2 gelangt.

Zur Beaufschlagung des Hand- oder Winkelstücks 1" mit einem oder mehrere Kühlmedien ist dieses mittels eines Versorgungsschlauchs 16 und darin angeordneten Fluidleitungen 17, 18 und 19 mit der Steuereinheit 28 verbindbar. Die Steuereinheit 28 ist hierbei bevorzugt ausgebildet den Fluidkreislauf 26" des Hand- oder Winkelstücks 1" mit einer Medienquelle und einen Medienauslass zu verbinden, so dass das Hand- oder Winkelstück 1" und die Antriebseinheit 4 von einem Kühlmedium durchströmbar ist.

Gemäß dem Ausführungsbeispiel sind die Fluidleitungen 17 und 19 des Versorgungsschlauchs 16 zum Zuführen zweier Kühlmedien mit einer Medienquelle verbunden. Die Fluidleitung 18 dient hierbei zum Rückführen des Kühlmediums und ist daher mit einem Medienauslass gekoppelt. Alternativ ist es auch möglich, dass die Fluidleitungen 17 und 19 zur Rückführung des Kühlmediums dienen und die Fluidleitung 18 mit dem zumindest einen Kühlmedium von der Steuereinheit 28 beaufschlagt wird.

Zur Kühlung der Behandlungsstelle und des Werkzeugs 2 umfasst die Behandlungsvorrichtung 27 bevorzugt des Weiteren eine Außenkühlung. Hierbei erstreckt sich eine externe Fluidleitung von der Steuereinheit 28, insbesondere von einer daran angeordneten Medienquelle, entlang der Außenseite des Versorgungsschlauchs 16 und/ oder des Hand- oder Winkelstücks 1" bis zu dessen Handstückkopf und somit bis zu dem in dem Hand- oder Winkelstück 1" aufgenommenen Werkzeug 2.

Damit die über den Versorgungsschlauch 16 mit der Steuereinheit 28 verbundenen Antriebseinheit 4 und die Steuereinheit 28 selbst auch mit Hand- oder Winkelstücken verwendbar sind, bei denen die Kühlmedien durch die Hand- oder Winkelstücke geführt werden und im Kopfbereich aus den Hand- oder Winkelstücken austreten, ist die Steuereinheit 28 bevorzugt ausgebildet alle drei Fluidleitungen 17, 18, 19 des Versorgungsschlauchs 16 und alle drei Fluidleitungen 20, 21, 22 der Antriebseinheit 4 mit einem oder mehrere Kühlmedien zu beaufschlagen.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Medizinisches, insbesondere zahnärztliches, Hand- oder Winkelstück (1, 1', 1") zum Antrieb eines medizinischen, insbesondere zahnärztlichen, Werkzeugs (2), aufweisend, ein erstes Kupplungselement (3) zur bevorzugt lösbaren Verbindung des Hand- oder Winkelstücks (1, 1', 1") mit einer Antriebseinheit (4) zur Übertragung einer Antriebsbewegung und eines Kühlmediums, eine Werkzeugaufnahme (5) zum bevorzugt lösbaren Anschließen des medizinischen, insbesondere zahnärztlichen, Werkzeugs (2) sowie einen Antriebsstrang (6) zur Übertragung einer Antriebsbewegung von der Antriebseinheit (4) auf die Werkzeugaufnahme (5), welcher zumindest eine in Rotation und/ oder Schwingung versetzbare Welle (7) umfasst, wobei das erste Kupplungselement (3) zur Kühlung des Hand- oder Winkelstücks (1, 1', 1") zumindest einen ersten Fluideinlass (8) und zumindest einen ersten Fluidauslass (9, 9', 9") umfasst, **dadurch gekennzeichnet, dass** der zumindest eine erste Fluideinlass (8) und der zumindest eine erste Fluidauslass (9, 9', 9") durch zumindest eine erste sich durch das Hand- oder Winkelstück (1, 1', 1") erstreckende Fluidleitung (10, 10', 10") verbunden sind, so dass ein Kühlmedium von dem zumindest einen ersten Fluideinlass (8) durch die Fluidleitung (10, 10', 10") zu dem zumindest einen ersten Fluidauslass (9, 9', 9") förderbar ist.

2. Medizinisches, insbesondere zahnärztliches, Hand- oder Winkelstück (1") nach Anspruch 1, **dadurch gekennzeichnet, dass** das Hand- oder Winkelstück (1") zumindest eine zweite sich durch das Hand- oder Winkelstück (1") erstreckende Fluidleitung (11) mit zumindest einem zweiten Fluideinlass (12) und zumindest einem zweiten Fluidauslass (13) umfasst, wobei der zumindest eine zweite Fluideinlass (12) und der zumindest eine zweite Fluidauslass (13) an dem ersten Kupplungselement (3) und/ oder an der zumindest einen ersten Fluidleitung (10") des medizinischen Hand- oder Winkelstücks (1") angeordnet sind.

3. Medizinisches, insbesondere zahnärztliches, Hand- oder Winkelstück (1") nach Anspruch 2, **dadurch gekennzeichnet, dass** die erste und/ oder zweite Fluidleitung (10", 11) zumindest ein Rückschlagventil (14) umfasst, so dass ein erstes und zweites Kühlmedium in der ersten und zweiten Fluidleitung (10", 11) nicht in die jeweils andere Fluidleitung (10", 11) des Hand- oder Winkelstücks (1") gelangt.

4. Medizinisches, insbesondere zahnärztliches, Hand- oder Winkelstück (1, 1', 1") nach einem der vorherigen Ansprüche, **dadurch gekennzeichnet, dass** die zumindest eine erste oder zweite Fluidleitung (10, 10', 10", 11) durch ein Leitungsrohr, ein den Antriebsstrang aufnehmendes Gehäuse (15), eine Bohrung in dem Gehäuse (15) und/ oder durch das erste Kupplungselement (3) gebildet ist.

5. Medizinisches, insbesondere zahnärztliches, Hand- oder Winkelstück (1, 1', 1") nach einem der vorherigen Ansprüche, **dadurch gekennzeichnet, dass** die zumindest eine erste oder zweite Fluidleitung (10, 10', 10", 11) hermetisch abgeschlossen ist, so dass das erste oder zweite Kühlmedium nicht aus dem Hand- oder Winkelstück (1, 1', 1") austritt.

6. Medizinisches, insbesondere zahnärztliches, Hand- oder Winkelstück (1") nach einem der vorherigen Ansprüche, **dadurch gekennzeichnet, dass** die erste und/ oder zweite Fluidleitung (10", 11) die Werkzeugaufnahme (5) des Hand- oder Winkelstücks (1") zumindest teilweise umgibt, so dass diese kühlbar ist.

7. Medizinisches, insbesondere zahnärztliches, Hand- oder Winkelstück (1', 1") nach einem der vorherigen Ansprüche, **dadurch gekennzeichnet, dass** das Hand- oder Winkelstück (1', 1") zumindest eine bevorzugt lösbar mit dem Hand- oder Winkelstück (1', 1") verbindbare Antriebseinheit (4) aufweist, welche mit einem Versorgungsschlauch (16) zur Übertragung von Daten und/ oder Energie sowie zumindest einem Kühlmedium verbindbar ist und zumindest eine erste durch die Antriebseinheit (4) verlaufende Fluidleitung (20) und zumindest eine zweite von der ersten Fluidleitung (20) getrennte Fluidleitung (21) umfasst, wobei die erste und zweite Fluidleitung (20, 21) der Antriebseinheit (4) über den zumindest einen ersten Fluideinlass (8) und den zumindest einen ersten Fluidauslass (9', 9") des Hand- oder Winkelstücks (1', 1") mit der zumindest einen ersten Fluidleitung (10', 10") des Hand- oder Winkelstücks (1', 1") verbindbar sind, so dass ein Kühlmedium von der ersten Fluidleitung (20) der Antriebseinheit (4) über die zumindest eine erste Fluidleitung (10', 10") des Hand- oder Winkelstücks (1', 1") in die zweite Fluidleitung (21, 22) der Antriebseinheit förderbar ist.

8. Medizinisches, insbesondere zahnärztliches, Hand- oder Winkelstück (1', 1") nach Anspruch 7, **dadurch gekennzeichnet, dass** die Antriebseinheit (4) ein zweites Kupplungselement (23) aufweist, welches bevorzugt lösbar mit dem ersten Kupplungselement (3) des Hand- oder Winkelstücks (1', 1") verbindbar ist, wobei eines der beiden Kupplungselemente (3) als Kupplungsausnehmung (24) ausgebildet ist, in die ein Kupplungsvorsprung (25) des anderen Kupplurigselements (23) einsetzbar ist.

9. Medizinisches, insbesondere zahnärztliches, Hand- oder Winkelstück (1', 1") nach Anspruch 8, **dadurch gekennzeichnet, dass** der zumindest eine erste Fluideinlass (8) und der zumindest eine erste Fluidauslass (9', 9") des Hand- oder Winkelstücks (1', 1") in dem Kupplungsvorsprung (25) oder der Kupplungsausnehmung (24) angeordnet sind.

10. Medizinisches, insbesondere zahnärztliches, Hand- oder Winkelstück (1', 1") nach einem der Ansprüche 7 bis 9, **dadurch gekennzeichnet, dass** die Antriebseinheit (4) einen Elektromotor mit einer Rotoreinheit, welche bevorzugt drehbar in einem Statorpaket gelagert ist, umfasst.

11. Medizinisches, insbesondere zahnärztliches, Hand- oder Winkelstück (1, 1', 1") nach einem der vorherigen Ansprüche, **dadurch gekennzeichnet, dass** der zumindest eine durch die mehreren Fluidleitungen (10, 10', 10"; 11, 21, 22, 23), Fluideinlässe (8, 12) und Fluidauslässe (9, 9', 9"; 13) gebildete Fluidkreislauf (26, 26', 26") hermetisch abgeschlossen ist, so dass das Kühlmedium nicht aus dem Hand- oder Winkelstück (1, 1', 1") und/ oder der Antriebseinheit (4) austritt.

12. Medizinisches, insbesondere zahnärztliches, Hand- oder Winkelstück (1, 1', 1") nach einem der vorherigen Ansprüche, **dadurch gekennzeichnet, dass** der Querschnitt der zumindest einen ersten oder zweiten Fluidleitung (10, 10', 10"; 11, 21, 22, 23) in Richtung des Fluidstromes ansteigt, so dass ein Rückstau des zumindest einen Kühlmediums in dem zumindest einen Fluidkreislauf (26, 26', 26") vermeidbar ist.

13. Medizinisches, insbesondere zahnärztliches, Hand- oder Winkelstück (1, 1', 1") nach einem der vorherigen Ansprüche, **dadurch gekennzeichnet, dass** die Fluideinlässe (8, 12) und die Fluidauslässe (9, 9', 9"; 13) der zumindest einen ersten oder zweiten Fluidleitung (10, 10', 10"; 11) des Hand- oder Winkelstücks (1, 1', 1") jeweils einen Regelungsabschnitt zur Regelung eines Fluidstroms aufweisen, so dass in einem entkoppelten Zustand der Kupplungselemente (3, 23) ein in den Fluidleitungen (10, 10', 10"; 11) verbleibendes Kühlmedium nicht aus dem Hand- oder Winkelstück (1, 1', 1") und/ oder der Antriebseinheit (4) strömbar ist.

14. Medizinisches, insbesondere zahnärztliches, Hand- oder Winkelstück (1, 1', 1") nach Anspruch 13, **dadurch gekennzeichnet, dass** der Regelungsabschnitt durch ein Rückschlagventil ausgebildet ist, welches einen Ventilsitz und einen Regelungskörper aufinreist.

15. Medizinische, insbesondere zahnärztliche, Behandlungsvorrichtung (27) zum Antrieb eines medizinischen, insbesondere zahnärztlichen, Werkzeugs (2), **gekennzeichnet durch** zumindest ein medizinisches, insbesondere zahnärztliches, Hand- oder Winkelstück (1, 1', 1") nach einem der Ansprüche 1 bis 14, zumindest einen mit dem Hand- oder Winkelstück (1, 1', 1") bevorzugt lösbar verbindbaren Versorgungsschlauch (16) zur Übertragung zumindest eines Kühlmediums, sowie zumindest eine Steuereinheit (28) zum Antreiben und/ oder Ansteuern des zumindest einen Hand- oder Winkelstücks (1, 1', 1"), welche dazu ausgebildet ist das Hand- oder Winkelstück (1, 1', 1") mit einer Medienquelle und einen Medienauslass zu verbunden, so dass das Hand- oder Winkelstück (1, 1', 1") von einem Kühlmedium durchströmbar ist.

## Claims

1. A medical, in particular dental, handpiece or contra-angle handpiece (1, 1', 1") for driving a medical, in particular dental tool (2), comprising a first coupling element (3) for a, preferably releasable, connection of the handpiece or contra-angle handpiece (1, 1', 1") to a drive unit (4) for transmitting a drive movement and a cooling medium, a tool receptacle (5) for a, preferably releasable, connection of the medical, in particular dental tool (2) and a drive train (6) for transmitting a drive movement from the drive unit (4) to the tool receptacle (5) which comprises at least one shaft (7) which can be induced to rotation and/or oscillation, wherein the first coupling element (3) comprises at least one first fluid inlet (8) and at least one first fluid outlet (9, 9', 9") for cooling the handpiece or contra-angle handpiece (1, 1', 1"), **characterized in that** the at least one first fluid inlet (8) and the at least one first fluid outlet (9, 9', 9") are connected by at least one first fluid line (10, 10', 10") extending through the handpiece or contra-angle handpiece (1, 1', 1") so that a cooling medium can be conveyed from the at least one first fluid inlet (8) through the fluid line (10, 10', 10") to the at least one first fluid outlet (9, 9', 9").

2. The medical, in particular dental, handpiece or contra-angle handpiece (1") according to claim 1, **characterized in that**
the handpiece or contra-angle handpiece (1") comprises at least one second fluid line (11) which extends through the handpiece or contra-angle handpiece (1") and has at least one second fluid inlet (12) and at least one second fluid outlet (13), wherein the at least one second fluid inlet (12) and the at least one second fluid outlet (13) are disposed on the first coupling element (3) and/or on the at least one first fluid line (10") of the medical handpiece or contra-angle handpiece (1").

3. The medical, in particular dental, handpiece or contra-angle handpiece (1") according to claim 2, **characterized in that**
the first and/or second fluid lines (10", 11) comprise at least one non-return valve (14), so that a first and a second cooling medium in the first and second fluid lines (10", 11) do not enter the other respective fluid line (10", 11) of the handpiece or contra-angle handpiece (1").

4. The medical, in particular dental, handpiece or contra-angle handpiece (1, 1', 1") according to any one of the preceding claims, **characterized in that** the at least one first or second fluid line (10, 10', 10", 11) is formed by a conduit, a housing (15) which accommodates the drive train, a bore in the housing (15) and/or by the first coupling element (3).

5. The medical, in particular dental, handpiece or contra-angle handpiece (1, 1', 1") according to any one of the preceding claims, **characterized in that** at least one first or second fluid line (10, 10', 10", 11) is hermetically sealed, so that the first or second cooling medium does not emerge from the handpiece or contra-angle handpiece (1, 1', 1").

6. The medical, in particular dental, handpiece or contra-angle handpiece (1") according to any one of the preceding claims, **characterized in that** the first and/or second fluid line(s) (10", 11) at least partially surrounds the tool receptacle (5) of the handpiece or contra-angle handpiece (1") so that it can be cooled.

7. The medical, in particular dental, handpiece or contra-angle handpiece (1, 1") according to any one of the preceding claims, **characterized in that** the handpiece or contra-angle handpiece (1', 1") comprises at least one drive unit (4) which can be connected, preferably releasably, to the handpiece or contra-angle handpiece (1', 1"), said drive unit can be connected to a supply tubing (16) for transmitting data and/or power and at least one cooling medium, and at least one first fluid line (20) which runs through the drive unit (4), and at least one second fluid line (21), which is separate from the first fluid line (20), wherein the first and second fluid lines (20, 21) of the drive unit (4) can be connected via the at least one first fluid inlet (8) and the at least one first fluid outlet (9', 9") of the handpiece or contra-angle handpiece (1', 1") to the at least one first fluid line (10', 10") of the handpiece or contra-angle handpiece (1', 1"), so that a cooling medium can be conveyed from the first fluid line (20) of the drive unit (4) through the at least one first fluid line (10', 10") of the handpiece or contra-angle handpiece (1', 1") into the second fluid line (21, 22) of the drive unit.

8. The medical, in particular dental, handpiece or contra-angle handpiece (1, 1") according to claim 7, **characterized in that**
the drive unit (4) comprises a second coupling element (23) which can be connected, preferably releasably, to the first coupling element (3) of the handpiece or contra-angle handpiece (1', 1"), wherein one of the two coupling elements (3) is designed as a coupling recess (24), into which a coupling protrusion (25) of the other coupling element (23) can be inserted.

9. The medical, in particular dental, handpiece or contra-angle handpiece (1, 1") according to claim 8, **characterized in that**
the at least one first fluid inlet (8) and the at least one first fluid outlet (9', 9") of the handpiece or contra-angle handpiece (1', 1") are arranged in the coupling protrusion (25) or in the coupling recess (24).

10. The medical, in particular dental, handpiece or contra-angle handpiece (1, 1") according to any one of claims 7 to 9, **characterized in that**
the drive unit (4) comprises an electric motor with a rotor unit which preferably is mounted rotatabely in a stator assembly.

11. The medical, in particular dental, handpiece or contra-angle handpiece (1, 1', 1") according to any one of the preceding claims, **characterized in that**
the at least one fluid circulation (26, 26', 26") formed by the plurality of fluid lines (10, 10', 10"; 11, 21, 22, 23), fluid inlets (8, 12) and fluid outlets (9, 9', 9"; 13) is hermetically sealed, so that the cooling medium does not escape from the handpiece or contra-angle handpiece (1, 1', 1") and/or the drive unit (4).

12. The medical, in particular dental, handpiece or contra-angle handpiece (1, 1', 1") according to any one of the preceding claims, **characterized in that**
the cross section of the at least one first or second fluid line (10, 10', 10"; 11, 21, 22, 23) increases in the direction of fluid flow so that a backing-up of the at least one cooling medium in the at least one fluid circulation (26, 26', 26") can be prevented.

13. The medical, in particular dental, handpiece or contra-angle handpiece (1, 1', 1") according to any one of the preceding claims, **characterized in that** the fluid inlets (8, 12) and the fluid outlets (9, 9', 9"; 13) of the at least one first or second fluid line (10, 10', 10"; 11) of the handpiece or contra-angle handpiece (1, 1', 1") each has/have a regulating section for regulating a fluid flow, so that in an uncoupled state of the coupling elements (3, 23) any cooling medium remaining in the fluid lines (10, 10', 10"; 11) cannot flow out of the handpiece or contra-angle handpiece (1, 1', 1") and/or drive unit (4).

14. The medical, in particular dental, handpiece or contra-angle handpiece (1, 1', 1") according to claim 13, **characterized in that**
the regulating section is formed by a non-return valve comprising a valve seat and a regulating body.

15. A medical, in particular dental, treatment device (27) for driving a medical, in particular dental, tool (2), **characterized by**
at least one medical, in particular dental, handpiece or contra-angle handpiece (1, 1', 1") according to any one of claims 1 to 14, at least one supply tubing (16) which can be connected, preferably releasably, to the handpiece or contra-angle handpiece (1, 1', 1") for transferring at least one cooling medium as well as at least one control unit (28) for driving and/or controlling the at least one handpiece or contra-angle handpiece (1, 1', 1"), which is designed to connect the handpiece or contra-angle handpiece (1, 1', 1") to a media source and a media outlet, so that a cooling medium can flow through the handpiece or contra-angle handpiece (1, 1', 1").

## Revendications

1. Pièce à main ou contre-angle (1, 1', 1") médicale, en particulier dentaire pour l'entraînement d'un outil (2) médical, en particulier dentaire, présentant un premier élément d'accouplement (3) pour la liaison de préférence détachable de la pièce à main ou du contre-angle (1, 1', 1") avec une unité d'entraînement (4) pour la transmission d'un mouvement d'entraînement et d'un réfrigérant, un raccordement d'outil (5) pour le raccordement de préférence détachable de l'outil (2) médical, en particulier dentaire ainsi qu'une chaîne cinématique (6) pour la transmission d'un mouvement d'entraînement de l'unité d'entraînement (4) sur le raccordement d'outil (5), laquelle comprend au moins un arbre (7) pouvant être mis en rotation et/ou vibration, dans lequel le premier élément d'accouplement (3) comprend, pour le refroidissement de la pièce à main ou du contre-angle (1, 1', 1''), au moins une première entrée de fluide (8) et au moins une première sortie de fluide (9, 9', 9''), **caractérisée en ce que**
l'au moins une première entrée de fluide (8) et l'au moins une première sortie de fluide (9, 9', 9'') sont reliées par au moins une première conduite de fluide (10, 10', 10'') s'étendant à travers la pièce à main ou le contre-angle (1, 1', 1'') de manière à ce qu'un réfrigérant puisse être acheminé depuis l'au moins une première entrée de fluide (8) à travers la conduite de fluide (10, 10', 10'') vers l'au moins une première sortie de fluide (9, 9', 9'').

2. Pièce à main ou contre-angle (1'') médicale, en particulier dentaire selon la revendication 1, **caractérisée en ce que**
la pièce à main ou le contre-angle (1'') comprend au moins une deuxième conduite de fluide (11) s'étendant à travers la pièce à main ou le contre-angle (1'') avec au moins une deuxième entrée de fluide (12) et au moins une deuxième sortie de fluide (13), dans laquelle l'au moins une deuxième entrée de fluide (12) et l'au moins une deuxième sortie de fluide (13) sont disposées au niveau du premier élément d'accouplement (3) et/ou au niveau de l'au moins une première conduite de fluide (10'') de la pièce à main ou du contre-angle (1'').

3. Pièce à main ou contre-angle (1'') médicale, en particulier dentaire selon la revendication 2, **caractérisée en ce que**
la première et/ou deuxième conduite de fluide (10'', 11) comprennent au moins une soupape anti-retour (14) de manière à ce qu'un premier et deuxième réfrigérant dans la première et deuxième conduite de fluide (10'', 11) ne parvienne pas dans l'autre conduite de fluide (10'', 11) respective de la pièce à main ou du contre-angle (1'').

4. Pièce à main ou contre-angle (1, 1', 1'') médicale, en particulier dentaire selon l'une des revendications précédentes, **caractérisée en ce que**
l'au moins une première ou deuxième conduite de fluide (10, 10', 10'', 11) est formée par un tuyau de conduite, un boîtier (15) réceptionnant la chaîne cinématique, un perçage dans le boîtier (15) et/ou par le premier élément d'accouplement (3).

5. Pièce à main ou contre-angle (1, 1', 1'') médicale, en particulier dentaire selon l'une des revendications précédentes, **caractérisée en ce que**
l'au moins une première ou deuxième conduite de fluide (10, 10', 10'', 11) est hermétiquement fermée de manière à ce que le premier ou deuxième réfrigérant ne sorte pas de la pièce à main ou du contre-angle (1, 1', 1'') .

6. Pièce à main ou contre-angle (1'') médicale, en particulier dentaire selon l'une des revendications précédentes, **caractérisée en ce que**
la première et/ou deuxième conduite de fluide (10'', 11) entoure au moins partiellement le raccordement d'outil (5) de la pièce à main ou du contre-angle (1'') de manière à pouvoir refroidir celui-ci.

7. Pièce à main ou contre-angle (1', 1'') médicale, en particulier dentaire selon l'une des revendications précédentes, **caractérisée en ce que**
la pièce à main ou le contre-angle (1', 1'') présente au moins une unité d'entraînement (4) pouvant être reliée de préférence de manière détachable à la pièce à main ou le contre-angle (1', 1''), laquelle peut être reliée à un tuyau d'alimentation (16) pour la transmission de données et/ou d'énergie ainsi que d'au moins un réfrigérant et comprend au moins une première conduite de fluide (20) s'étendant à travers l'unité d'entraînement (4) et au moins une deuxième conduite de fluide (21) séparée de la première conduite de fluide (20), dans laquelle les première et deuxième conduites de fluide (20, 21) de l'unité d'entraînement (4) peuvent être reliées via l'au moins une première entrée de fluide (8) et l'au moins une première sortie de fluide (9', 9'') de la pièce à main ou du contre-angle (1', 1'') à l'au moins une première conduite de fluide (10', 10'') de la pièce à main ou du contre-angle (1', 1'') de manière à ce qu'un réfrigérant puisse être acheminé depuis la première conduite de fluide (20) de l'unité d'entraînement (4) via l'au moins une première conduite de fluide (10', 10'') de la pièce à main ou du contre-angle (1', 1'') jusque dans la deuxième conduite de fluide (21, 22) de l'unité d'entraînement.

8. Pièce à main ou contre-angle (1', 1") médicale, en particulier dentaire selon la revendication 7, **caractérisée en ce que**
l'unité d'entraînement (4) présente un deuxième élément d'accouplement (23) qui peut être relié de préférence de manière détachable au premier élément d'accouplement (3) de la pièce à main ou du contre-angle (1', 1"), dans laquelle l'un des deux éléments d'accouplement (3) est réalisé en tant qu'évidement d'accouplement (24) dans lequel il est possible de placer une saillie d'accouplement (25) de l'autre élément d'accouplement (23).

9. Pièce à main ou contre-angle (1', 1") médicale, en particulier dentaire selon la revendication 8, **caractérisée en ce que**
l'au moins une première entrée de fluide (8) et l'au moins une première sortie de fluide (9', 9") de la pièce à main ou du contre-angle (1', 1") sont disposées dans la saillie d'accouplement (25) ou dans l'évidement d'accouplement (24).

10. Pièce à main ou contre-angle (1', 1'') médicale, en particulier dentaire selon l'une des revendications 7 à 9, **caractérisée en ce que**
l'unité d'entraînement (4) comprend un moteur électrique avec une unité de rotor qui est de préférence montée de manière rotative dans un paquet statorique.

11. Pièce à main ou contre-angle (1, 1', 1") médicale, en particulier dentaire selon l'une des revendications précédentes, **caractérisée en ce que**
l'au moins un circuit de fluide (26, 26', 26'') formé par les plusieurs conduites de fluide (10, 10', 10" ; 11, 21, 22, 23), entrées de fluide (8, 12) et sorties de fluide (9, 9', 9" ; 13), est hermétiquement fermé de manière à ce que le réfrigérant ne sorte pas de la pièce à main ou du contre-angle (1, 1', 1'') et/ou de l'unité d'entraînement (4).

12. Pièce à main ou contre-angle (1, 1', 1") médicale, en particulier dentaire selon l'une des revendications précédentes, **caractérisée en ce que**
la section transversale de l'au moins une première ou deuxième conduite de fluide (10, 10', 10" ; 11, 21, 22, 23) augmente en direction du flux de fluide de manière à éviter un refoulement de l'au moins un réfrigérant dans l'au moins un circuit de fluide (26, 26', 26'').

13. Pièce à main ou contre-angle (1, 1', 1") médicale, en particulier dentaire selon l'une des revendications précédentes, **caractérisée en ce que**
les entrées de fluide (8, 12) et les sorties de fluide (9, 9', 9" ; 13) de l'au moins une première ou deuxième conduite de fluide (10, 10', 10" ;11) de la pièce à main ou du contre-angle (1, 1', 1") présentent respectivement une section de régulation pour la régulation d'un flux de fluide de manière à ce que, dans un état découplé des éléments d'accouplement (3, 23), un réfrigérant subsistant dans les conduites de fluide (10, 10', 10" ; 11) ne puisse pas s'écouler hors de la pièce à main ou du contre-angle (1, 1', 1") et/ou de l'unité d'entraînement (4).

14. Pièce à main ou contre-angle (1, 1', 1") médicale, en particulier dentaire selon la revendication 13, **caractérisée en ce que**
la section de régulation est formée par une soupape antiretour qui présente un siège de soupape et un corps de régulation.

15. Dispositif de traitement (27) médical, en particulier dentaire, pour l'entraînement d'un outil (2) médical, en particulier dentaire, **caractérisé par**
au moins une pièce à main ou contre-angle (1, 1', 1") médicale, en particulier dentaire selon l'une des revendications 1 à 14, au moins un tuyau d'alimentation (16) pouvant être relié de préférence de façon détachable à la pièce à main ou le contre-angle (1, 1', 1'') pour la transmission d'au moins un réfrigérant, ainsi qu'au moins une unité de commande (28) pour l'entraînement et/ou la commande de l'au moins une pièce à main ou contre-angle (1, 1', 1"), laquelle est étudiée pour relier la pièce à main ou le contre-angle (1, 1', 1'') à une source de milieu et une sortie de milieu de manière à ce que la pièce à main ou le contre-angle (1, 1' , 1") puisse être traversée par un réfrigérant.
